# EUROPEAN PATENT SPECIFICATION

(11) **EP 0 683 764 B1**
(45) Date of publication and mention of the grant of the patent: **17.09.1997**
(21) Application number: 94907533.7
(22) Date of filing: 07.02.1994
(51) Int. Cl.: C07C 67/38, B01J 31/24, C07C 69/24

(54) **CARBONYLATION PROCESS**
CARBONYLIERUNGSVERFAHREN
PROCEDE DE CARBONYLATION

(30) Priority: 09.02.1993 EP 93200360
(43) Date of publication of application: 29.11.1995
(73) Proprietor: SHELL INTERNATIONALE RESEARCH MAATSCHAPPIJ B.V., 2596 HR Den Haag (NL)
(72) Inventor: DRENT, Eit, NL-1031 CM Amsterdam (NL); PELLO, Dennis, Humphrey, Louis, NL-1031 CM Amsterdam (NL); HASSELAAR, Melis, NL-1031 CM Amsterdam (NL)
(86) International application number: PCT/EP94/00379
(87) International publication number: WO 94/18154

(56) References cited:
- EP-A- 0 055 875
- EP-A- 0 441 446
- EP-A- 0 495 547

## Description

The invention relates to a process for the carbonylation of ethylenically unsaturated compounds by reaction with carbon monoxide and a co-reactant in the presence of a palladium-containing catalyst system.

Various carbonylation reactions of this type are disclosed in EP-A-0,495,547. In the process described in that document, an optionally substituted olefinically unsaturated compound is reacted with carbon monoxide and a variety of co-reactants in the presence of a catalyst system comprising: a source of palladium cations; a source of bidentate ligands; and a source of anions, wherein the ligands are selected from diphosphines having the general formula Q¹Q²PXPQ³Q⁴, wherein the groups Q¹, Q², Q³, and Q⁴ independently represent an optionally substituted aliphatic group, or Q¹ together with Q², and/or Q³ together with Q⁴, represent an optionally substituted bivalent aliphatic group, and X represents a bivalent bridging group containing from 1 to 10 atoms in the bridge. As anion source, preferably a protonic acid is selected. Preference is in particular given to sources of non- or weakly-coordinating anions, i.e. anions which do not or only weakly coordinate with the palladium cation.

Whilst the process disclosed in EP-A-0,495,547 in general provides the desired product in good yield and with high selectivity, it has been experienced that the reaction rate in some instances leaves room for improvement. Efforts have therefore continued to achieve an increase in reaction rate, without impeding other factors such as selectivity or catalyst stability.

Surprisingly it has now been found, that by using a substantially non-acidic catalyst system and by selecting a specific category of bidentate ligands, the rate of a number of carbonylation reactions is significantly increased.

The invention may be defined as relating to a process for the carbonylation of ethylenically unsaturated compounds by reaction with carbon monoxide and a co-reactant in the presence of a substantially non-acidic catalyst system based on a palladium compound and a bidentate ligand of the formula

R¹R²M¹RM²R³R⁴ (I)

wherein M¹ and M² independently represents a phosphorus, arsenic or antimony atom, R represents a bivalent organic bridging group, R¹, R², R³ and R⁴ independently represent substituted or non-substituted aliphatic groups, with the proviso that R¹ together with R², and/or R³ together with R⁴ represent a bivalent cyclic group with at least 5 ring atoms whereby the two free valencies are linked to M¹ or M², respectively.

It is believed that the carbonylation reactions according to the invention proceed under the influence of an active catalyst system containing palladium cations, in complex coordination with a bidentate ligand.

The palladium cations may originate from salts, for example salts derived from nitric acid, sulphuric acid, and sulphonic acids, such as p-toluenesulphonic acid, methanesulphonic acid or trifluoromethanesulphonic acid. Preferably, a palladium salt of a carboxylic acid is used, for example acetic acid, trifluoroacetic acid or propionic acid. If desired, the metallic element or a zero valent palladium complex, e.g. with carbon monoxide can be used. However, this will require the presence of a protonic acid and it will be appreciated that the amount thereof has to be selected very carefully, in order to avoid that an acidic catalyst system results.

In the bidentate ligands of formula (I), both M¹ and M² preferably represent phosphorus atoms. R preferably represents a bivalent organic bridging group, containing from 1 to 10 atoms in the bridge. More preferably, R represents an alkylene group containing from 1 to 4 atoms in the bridge. In general the bridging group consists of carbon and hydrogen atoms only, but it may also comprise a carbon chain, interrupted by a heteroatom, such as an oxygen atom. In particular preferred are ligands of formula (I) in which R represents an ethylene group.

The cyclic group(s) formed by R¹ together with R², and/or R³ together with R⁴ comprise at least 5 ring atoms, in particular from 6 to 9 ring atoms. As a rule they comprise only carbon atoms as ring atoms, but cyclic groups containing 1 or 2 heteroatoms in the ring, such as oxygen- or nitrogen-ring atoms are not precluded. Very suitable are bivalent cyclic groups, whereby the ring consists of 7 or, preferably, 8 carbon atoms. The two free valencies may occur at adjacent carbon ring atoms, or at two carbon atoms which are further apart. Examples of suitable cyclic groups are: 1,2-cyclooctylene, 1,3-cyclooctylene, 1,4-cyclooctylene, 1,5-cyclooctylene, 1,4-cyclohexylene, 1,3-cycloheptylene and 1,4-cycloheptylene groups. Mixtures of bidentate ligands may be used as well, whereby R¹ together with R² represent various cyclic groups and R³ and R⁴ represent either non-cyclic aliphatic groups or R³, together with R⁴ represent cyclic groups which may be the same as or different from the cyclic group represented by R¹ and R². Examples of suitable bidentate ligands selected from the [4.2.1] and/or [3.3.1] isomers of 1,2-bis(9-phosphabicyclononyl)ethane and 1,2-bis(9-phosphabicyclononyl)propane; 1,3-bis(9-phosphabicyclo-[3.3.1.]nonyl)propane and 1,3-bis(9-phosphabicyclononyl)propane; and 1-dialkylphosphino-2-P-(9-phosphabicyclononyl)ethane. If R¹ and R², or R³ and R⁴ do not represent a bivalent cyclic group, they suitably represent an optionally substituted alkyl, preferably a C₁₋₆ alkyl, or monovalent cycloalkyl, preferably a cyclo C₅₋₁₀ alkyl group. Examples of suitable alkyl groups are methyl, ethyl or butyl groups. Suitable cycloalkyl groups are for example cyclohexyl or cyclooctyl groups.

The ratio of number of moles of ligands of formula (I) per gram atom of palladium is preferably in the range of from 0.5 to 10, most preferably in the range of from 1 to 3.

The process according to the invention is preferably carried out in the presence of a basic compound. It is believed that the presence of a basic compound not only ensures that the catalyst system remains non-acidic during the process, but also favourably affects the catalytic activity. Accordingly, it is considered likely, that at least part of the quantity of basic compound participates in the catalyst system. Suitable basic compounds, in particular, are nitrogen bases, preferably nitrogen-containing compounds wherein the nitrogen atom(s) is (are) only linked to atoms, other than hydrogen. Examples of suitable nitrogen bases are trialkylamines, preferably tri-C₁₋₆ alkyl-amines, especially triethylamine, tripropylamine, tri-n-butylamine and tri-tert.butylamine. Examples of suitable cyclic amines are pyridine and alkylpyridines, e.g. (C₁₋₄ alkyl)pyridines, such as 2-methylpyridine, 3,4-dimethylpyridine and 1,10-phenanthroline. The amount of basic compound is not critical and may vary between wide limits. Usually a molar amount of basic compound is selected in the range of from 1 to 100, preferably in the range of from 1 to 20, per gram atom of palladium. Conveniently, the amount of catalyst system is relatively small. Preferred amounts are in the range of 10⁻⁷ to 10⁻¹ gram atom palladium per mole of ethylenically unsaturated compound, more preferably in the range of 10⁻⁶ to 10⁻² on the same basis.

Catalyst compositions comprising:
a) a palladium compound;
b) a bidentate ligand of the formula

   R¹R²PRPR³R⁴ (II)

   wherein R is a bivalent organic bridging group containing from 1 to 10 atoms in the bridge and R¹, R², R³ and R⁴ independently represent substituted or non-substituted aliphatic groups, with the proviso that R¹ together with R² and/or R³ together with R⁴ represent a bivalent cyclic group with at least 6 ring atoms, whereby the two free valencies are linked to the phosphorus atom(s); and
c) a basic compound,
are novel.

Accordingly, the invention also relates to these novel catalyst compositions. The invention in particular relates to catalyst compositions comprising: a palladium compound; a bidentate ligand of formula (II), wherein R¹R²P and R³R⁴P each represent the [4.2.1] and/or [3.3.1] isomer of a 9-phosphabicyclononyl group and R represents an ethylene group; and pyridine or a trialkylamine.

The bidentate ligands of formula (II) may be prepared by known techniques such as those described in British Patent Specification No. 1,127,965.

The ethylenically unsaturated compound may have one or more double bonds. It is preferably an olefin having from 2 to 20 carbon atoms per molecule. The unsaturated bond(s) may be internal or, preferably, terminal. In particular preferred are olefins having from 2 to 8 carbon atoms per molecule, such as ethene, propene, butene-1, hexene-1 and octene-1.

In the ethylenically unsaturated compound one or more hydrogen atoms may have been substituted by other atoms, such as halogen atoms or by groups of atoms, such as hydroxyl groups, cyano groups, such as methoxy or ethoxy groups, or amino groups such as dimethyl- and diethyl-amino groups.

Another preferred category of ethylenically unsaturated compounds, consists of unsaturated esters of carboxylic acids and esters of unsaturated carboxylic acids. For example, the starting material may be a vinyl ester of a carboxylic acid such as acetic acid or propionic acid, or it may be an alkyl ester of an unsaturated acid, such as the methyl or ethyl ester of acrylic acid or methacrylic acid.

Suitable co-reactants in the process of the invention include compounds comprising a nucleophilic moiety and a mobile hydrogen atom. Examples are mono- and dihydric alkanols, such as methanol, ethanol, n-butanol, ethylene glycol, isopropanol, butanediols and hexanol-1, and amines, such as ethylamine and diethylamine. Alkanols with 1 to 6 carbon atoms per molecule and alkanediols with 2 to 6 carbon atoms per molecule are preferred. n-Butanol-1, methanol and 1,4-butanediol are especially preferred. The use of these compounds as co-reactants enables the production of valuable carbonylation products, such as methylpropionate, butylpropionate and 1,4-diacyloxy butanes.

These compounds are of commercial interest and may be used as solvents and in flavouring compositions and perfumes. In the process of the invention the ethylenically unsaturated compound, or the co-reactant may be used in excess and, accordingly, may serve as solvent during the reaction. It is also possible to perform the reaction in the presence of an additional liquid diluent, in particular when the reactants are used in stoichiometric amounts. Suitable diluents are for example polar, aprotic compounds, such as ketones or ethers. Preferred diluents are tetrahydrofuran and the dimethylether of diethyleneglycol (diglyme).

If so desired, the reaction may be performed in the further presence of a promoter, such as a drying agent. Suitable drying agents include acetals, such as dimethyl-acetal of acetone, ketals and the like. A preferred drying agent is trimethyl orthoformate.

The carbonylation reaction is conveniently carried out at moderate temperatures, generally being in the range of from 30 to 200 °C, preferably in the range of from 50 to 150 °C. Reaction pressures may be atmospheric or superatmospheric. In particular pressures in the range of from 5 to 70 bar are preferred. Higher pressures are not precluded, but usually do not provide advantages.

The invention will be illustrated by the following examples.

### Examples I-XI

The experiments were carried out by each time charging a 250 mL magnetically-stirred "Hastelloy C" (trade mark) autoclave with 0.25 mmol palladium(II)acetate, 0.6 mmol of a diphosphine ligand, 50 mL of an alkanol co-reactant and, where applicable, one or more additives. The autoclave was flushed with a 1.5:1 molar mixture of carbon monoxide and ethene and pressurized to a total pressure of 50 bar. The autoclave was then sealed and the mixture was heated to the desired reaction temperature. The reaction was continued to complete ethene conversion and subsequently the reaction mixture was cooled to room temperature and the pressure released. The ligand, co-reactant, additive (if any), reaction temperature and average hourly rate of ester formation are shown in Table 1.

The abbreviations used in the table have the following meanings:
- BPBNP =: 1,3-bis(9-phospha-bicyclononyl)propane
- MBPBNE =: 1-methyl-1,2-bis(9-phospha-bicyclononyl)ethane
1,2-bis(9-phosphabicyclononyl)propane
- BPBNE =: 1,2-bis(9-phospha-bicyclononyl)ethane
- DMP =: 3,4-dimethylpyridine(lutidine)
- Phen =: 1,10-phenanthroline
- BBA =: 2,6-di(sec.butoxy)benzoic acid
- TEA: = triethylamine
- TMF =: trimethyl orthoformate

The product obtained in Example V was methylpropionate; in the other examples n-butylpropionate was obtained. The selectivity with respect to the ester was more than 99%. From the results of Examples I, II and III it can be seen that by the presence of a basic compound, the rate is increased and that a further increase in rate is obtained by adding a drying agent. Likewise, comparing the results of Examples IV, VI, VII and VIII, an increase in rate is observed by adding a drying agent or a basic compound. The effect of adding a drying agent is further illustrated by comparing the results of Examples IX and X.

### Example B (not according to the invention, for comparison only)

An experiment was carried out, substantially under the conditions as described for Example IV with the differences that the reaction temperature was 130 °C, instead of 125 °C and that as ligand 1,2-bis(dicyclohexylphosphino)ethane was used. The rate was 30 mol/gat Pd.h.

### Example C (not according to the invention, for comparison only)

An experiment was carried out, substantially under the conditions as described for Example II, with the difference that as ligand 1,3-bis(di-sec.butylphosphino)propane was used. The rate was 50 mol/gat Pd.h.

**Table 1**

| Example | Ligand | Additive (mmol) | Alkanol (mL) | Temperature (° C) | Rate (mol/gat Pd.h) |
|---|---|---|---|---|---|
| I | BPBNP | - | n-butanol 50 | 125 | 35 |
| II | " | DMP 10 | " | 137 | 85 |
| III | " | " | " | 137 | 330 |
| | | TMF 5 mL | | | |
| IV | BPBNE | - | " | 125 | 520 |
| V | " | - | methanol 50 | 125 | 490 |
| VI | " | TMF 1 mL | n-butanol 50 | 125 | 600 |
| VII | " | Phen 1 | " | 125 | 630 |
| VIII | " | Phen 2.5 | " | 125 | 1150 |
| | | TMF 1 mL | | | |
| A* | " | BBA 10 | " | 120 | 360 |
| IX | " | TEA 10 | " | 133 | 275 |
| X | " | " | " | 133 | 725 |
| | | TMF 5 mL | | | |
| XI | " | DMP 10 | " | 135 | 1160 |
| | | TMF 5 mL | | | |

| | | | | | |
|---|---|---|---|---|---|
| * not according to the invention, for comparison only | | | | | |

### Example XII

An experiment was carried out, whereby the autoclave (magnetically stirred, 250 mL, "Hastelloy C") was charged with 0.25 mmol palladium (II) acetate, 0.6 mmol of a diphosphine ligand (BPBNP as defined above), 10 mmol of triethylamine, 5 mL of trimethyl orthoformate, 50 mL of methanol and 20 mL of n-octene-1. The autoclave was flushed and pressurized with carbon monoxide to a pressure of 30 bar. The mixture was heated to 130 °C. The reaction was continued to complete octene conversion and subsequently the reaction mixture was cooled to room temperature and the pressure released. The rate was 100 mol/gat Pd.h. The linearity of the formed methyl ester of nonanoic acid was 73%.

### Example XIII

An experiment was carried out, substantially as described in Example XII, with the difference that instead of BPBNP as ligand BPBNE was used, (BPBNP and BPBNE are as defined above). The rate was 170 mol/gat Pd.h. The linearity of the formed methyl ester of nonanoic acid was 69%.

### Example XIV

An experiment was carried out, substantially as described in Example I, with the difference that 40 instead 50 mL n-butanol was used, at a temperature of 130 °C and using MBPBNE instead of BPBNP as ligand, (BPBNP and MBPBNE are as defined above). The rate was 200 mol/gat Pd.h. The selectivity with respect to n-butylpropionate was more than 99%.

## Claims

1. A process for the carbonylation of ethylenically unsaturated compounds by reaction with carbon monoxide and a co-reactant in the presence of a substantially non-acidic catalyst system based on a palladium compound and a bidentate ligand of the formula
R¹R²M¹RM²R³R⁴ (I)
wherein
M¹ and M² independently represent a phosphorus, arsenic or antimony atom, R represents a bivalent organic bridging group, R¹,
R², R³ and R⁴ independently represent substituted or non-substituted aliphatic groups, with the proviso that R¹ together with R²,
and/or R³ together with R⁴ represent a bivalent cyclic group with at least 5 ring atoms whereby the two free valencies are linked to M¹ or M², respectively.

2. A process as claimed in claim 1, wherein M¹ and M² both represent phosphorus atoms.

3. A process as claimed in claim 1 or 2, wherein R represents a bivalent organic bridging group containing from 1 to 10 atoms in the bridge.

4. A process as claimed in any one of claims 1 to 3, wherein R represents an ethylene group.

5. A process as claimed in any one of claims 1 to 4, wherein the bivalent cyclic group represented by R¹ together with R², and/or by R³ together with R⁴, is a cycloalkylene group having from 6 to 9 ring atoms.

6. A process as claimed in claim 5, wherein R is linked to two phosphorus atoms, each of which participates in the [4.2.1] and/or [3.3.1] isomer of a 9-phosphabicyclononyl group.

7. A process as claimed in any one of claims 1 to 6, wherein the reaction is carried out in the presence of a basic compound.

8. A process as claimed in claim 7, wherein as basic compound pyridine, a substituted pyridine or a trialkylamine is used.

9. A process as claimed in any one of claims 1 to 8, wherein the reaction is carried out in the presence of a liquid diluent.

10. A process as claimed in claim 9, wherein as diluent an ether is used, in particular tetrahydrofuran or the dimethylether of diethylene glycol.

11. Catalyst composition comprising:
a) a palladium compound;
b) a bidentate ligand of the formula
R¹R²PRPR³R⁴ (II)
wherein R is a bivalent organic bridging group containing from 1 to 10 atoms in the bridge and R¹, R², R³ and R⁴ independently represent substituted or non-substituted aliphatic groups, with the proviso that R¹ together with R² and/or R³ together with R⁴ represent a bivalent cyclic group with at least 6 ring atoms whereby the two free valencies are linked to the phosphorus atom(s) and
c) a basic compound.

12. Catalyst composition as claimed in claim 11, wherein R¹R²P and R³R⁴P each represent the [4.2.1] and/or [3.3.1] isomer of a 9-phosphabicyclononyl group and wherein the basic compound is pyridine, a substituted pyridine or a trialkylamine.

## Patentansprüche

1. Verfahren zur Carbonylierung von ethylenisch ungesättigten Verbindungen durch Umsetzung mit Kohlenmonoxid und einem Coreaktanten in Anwesenheit eines im wesentlichen nichtsauren Katalysatorsystems auf der Basis einer Palladiumverbindung und eines Bidentatliganden mit der Formel
R¹R²M¹RM²R³R⁴ (I)
bezieht, worin M¹ und M² unabhängig voneinander ein Phosphor-, Arsen- oder Antimonatom darstellen, R eine zweiwertige organische Brückengruppe bedeutet, R¹, R², R³ und R⁴ unabhängig voneinander substituierte oder unsubstituierte aliphatische Gruppen bedeuten, mit der Maßgabe, daß R¹ zusammen mit R^{2,} und/oder R³ zusammen mit R⁴, eine zweiwertige cyclische Gruppe mit wenigstens 5 Ringatomen bedeuten, wobei die beiden freien Valenzen an M¹ bzw. M² geknüpft sind.

2. Verfahren nach Anspruch 1, worin sowohl M¹ als auch M² Phosphoratome darstellen.

3. Verfahren nach Anspruch 1 oder Anspruch 2, worin R eine zweiwertige organische Brückengruppe mit einem Gehalt an 1 bis 10 Atomen in der Brücke darstellt.

4. Verfahren nach einem der Ansprüche 1 bis 3, worin R eine Ethylengruppe darstellt.

5. Verfahren nach einem der Ansprüche 1 bis 4, worin die zweiwertige, durch R¹ zusammen mit R² und/oder durch R³ zusammen mit R⁴ dargestellte cyclische Gruppe eine Cycloalkylengruppe mit 6 bis 9 Ringatomen ist.

6. Verfahren nach Anspruch 5, worin R mit zwei Phosphoratomen verknüpft ist, wovon jedes Teil des [4.2.1.]- und/oder [3.3.1.]-Isomers einer 9-Phosphabicyclononylgruppe ist.

7. Verfahren nach einem der Ansprüche 1 bis 6, worin die Umsetzung in Anwesenheit einer basischen Verbindung ausgeführt wird.

8. Verfahren nach Anspruch 7, worin als basische Verbindung Pyridin, ein substituiertes Pyridin oder ein Trialkylamin verwendet wird.

9. Verfahren nach einem der Ansprüche 1 bis 8, worin die Umsetzung in Anwesenheit eines flüssigen Verdünnungsmittels ausgeführt wird.

10. Verfahren nach Anspruch 9, worin als Verdünnungsmittel ein Ether verwendet wird, insbesondere Tetrahydrofuran oder der Diethylenglycoldimethylether.

11. Katalysatorzusammensetzung mit einem Gehalt an:
a) einer Palladiumverbindung;
b) einem Bidentatliganden mit der Formel
R¹R²PRPR³R⁴ (II),
worin R eine zweiwertige organische Brückengruppe mit einem Gehalt an 1 bis 10 Atomen in der Brücke darstellt und R¹, R², R³ und R⁴ unabhängig voneinander substituierte oder unsubstituierte aliphatische Gruppen bedeuten, mit der Maßgabe, daß R¹ zusammen mit R² und/oder R³ zusammen mit R⁴ eine zweiwertige cyclische Gruppe mit wenigstens 6 Ringatomen bedeuten, wobei die beiden freien Valenzen an das Phosphoratom bzw. an die Phosphoratome geknüpft sind; und
c) einer basischen Verbindung.

12. Katalysatorzusammensetzung nach Anspruch 11, worin R¹R²P und R³R⁴P jeweils das [4.2.1]- und/oder [3,3,1]-Isomer einer 9-Phosphabicyclononylgruppe darstellen und worin die basische Verbindung Pyridin, ein substituiertes Pyridin oder ein Trialkylamin ist.

## Revendications

1. Procédé de carbonylation de composés éthyléniquement insaturés par réaction avec le monoxyde de carbone et un coréactif en présence d'un système catalytique sensiblement non acide, à base d'un composé du palladium et d'un ligand bidentate de la formule :
R¹R²M¹RM²R³R⁴ (I)
dans laquelle M¹ et M² représentent, chacun indépendamment, un atome de phosphore, d'arsenic ou d'antimoine, R représente un groupe de pontage organique bivalent, R¹, R², R³ et R⁴ représentent, chacun indépendamment, un radical aliphatique substitué ou non substitué, avec comme conditions que R¹ en même temps que R² et/ou R³ en même temps que R⁴ représentent un groupe cyclique bivalent avec au moins 5 atomes cycliques, où les deux valences libres sont liées à M¹ ou M², respectivement.

2. Procédé suivant la revendication 1, caractérisé en ce que M¹ et M² représentent tous deux des atomes de phosphore.

3. Procédé suivant la revendication 1 ou 2, caractérisé en ce que R représente un groupe de pontage organique bivalent contenant de 1 à 10 atomes dans le pont.

4. Procédé suivant l'une quelconque des revendications 1 à 3, caractérisé en ce que R représente le radical éthylène.

5. Procédé suivant l'une quelconque des revendications 1 à 4, caractérisé en ce que le radical cyclique bivalent représenté par R¹ ensemble avec R² et/ou par R³ ensemble avec R⁴, est un radical cycloalkylène possédant de 6 à 9 atomes cycliques.

6. Procédé suivant la revendication 5, caractérisé en ce que R est lié à deux atomes de phosphore, chacun d'entre eux participant à l'isomère [4.2.1] et/ou [3.3.1] d'un groupe 9-phosphabicyclononyle.

7. Procédé suivant l'une quelconque des revendications 1 à 6, caractérisé en ce que l'on entreprend la réaction en présence d'un composé basique.

8. Procédé suivant la revendication 7, caractérisé en ce que l'on utilise la pyridine, et une pyridine substituée, ou une trialkylamine, à titre de composé basique.

9. Procédé suivant l'une quelconque des revendications 1 à 8, caractérisé en ce que l'on entreprend la réaction en présence d'un diluant liquide.

10. Procédé suivant la revendication 9, caractérisé en ce qu'à titre de diluant, on utilise un éther, en particulier le tétrahydrofuranne ou l'éther diméthylique du diéthylèneglycol.

11. Composition catalytique qui comprend :
a) un composé du palladium;
b) un ligand bidentate de la formule :
R¹R²PRPR³R⁴ (II)
dans laquelle R représente un groupe de pontage organique bivalent contenant de 1 à 10 atomes dans le pont et R¹, R², R³ et R⁴ représentent, chacun indépendamment, un radical aliphatique substitué ou non substitué, avec la condition que R¹ ensemble avec R² et/ou R³ ensemble avec R⁴ représentent un groupe cyclique bivalent comportant au moins 6 atomes cycliques, où les deux valences libres sont liées à l'atome ou aux atomes de phosphore; et
c) un composé basique.

12. Composition catalytique suivant la revendication 11, caractérisée en ce que R¹R²P et R³R⁴P représentent chacun l'isomère [4.2.1] et/ou [3.3.1] d'un groupe 9-phosphabicyclononyle et en ce que le composé basique est la pyridine, une pyridine substituée ou une trialkylamine.
